# EUROPEAN PATENT APPLICATION

(11) **EP 3 734 286 A1**
(43) Date of publication of application: **04.11.2020**
(21) Application number: 20175031.2
(22) Date of filing: 15.05.2020
(51) Int. Cl.: G01N 33/68

(54) **A METHOD FOR DETERMINING THE EFFICACY OF A SARS-COV-2 VACCINE**

(71) Applicant: Euroimmun Medizinische Labordiagnostika AG, 23560 Lübeck (DE)
(72) Inventor: The designation of the inventor has not yet been filed
(74) Representative: Richter, Carsten

(57) **Abstract**

The present invention relates to method for determining whether a subject produces antibodies as a result of administration of a vaccine for distinguishing whether a subject produces antibodies as a result of administration of a vaccine or as a result of a previous corona virus infection, comprising the step detecting in a sample comprising antibodies from said subject the absence or presence of an antibody to SEQ ID NO1 and the absence or presence of an antibody to SEQ DI NO2.

SEQUENCE LISTING

<110>  EUROIMMUN Medizinische Labordiagnostika AG

<120>  A method for determining the efficacy of a vaccine

<130>  20PP069EP

<160>  4

<170>  PatentIn version 3.5

<210>  1
<211>  670
<212>  PRT
<213>  Severe acute respiratory syndrome coronavirus 2

<400>  1

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
        180                 185                 190

Thr Pro Ile Asn Leu Val Arg Asp Leu Pro Gln Gly Phe Ser Ala Leu
    195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr
465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg
            660                 665                 670

<210>  2
<211>  419

<212>  PRT
<213>  Severe acute respiratory syndrome coronavirus 2

<400>  2

Met Ser Asp Asn Gly Pro Gln Asn Gln Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Ala Arg Ser Lys Gln Arg Arg Pro Gln Gly Leu Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Pro
65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Ser Arg Asn
            180                 185                 190

Ser Ser Arg Asn Ser Thr Pro Gly Ser Ser Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Gln Leu Glu Ser Lys Met Ser Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Leu Ile Arg Gln Gly Thr Asp Tyr Lys His Trp Pro Gln Ile
    290                 295                 300

Ala Gln Phe Ala Pro Ser Ala Ser Ala Phe Phe Gly Met Ser Arg Ile
305                 310                 315                 320

Gly Met Glu Val Thr Pro Ser Gly Thr Trp Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro
        355                 360                 365

Lys Lys Asp Lys Lys Lys Lys Ala Asp Glu Thr Gln Ala Leu Pro Gln
    370                 375                 380

Arg Gln Lys Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  3
<211>  300
<212>  PRT
<213>  Artificial Sequence

<220>
<223>  SARS-CoV-2 nucleoprotein (118-419)

<400>  3

Gly Leu Pro Tyr Gly Ala Asn Lys Asp Gly Ile Ile Trp Val Ala Thr
1               5                   10                  15

Glu Gly Ala Leu Asn Thr Pro Lys Asp His Ile Gly Thr Arg Asn Pro
            20                  25                  30

Ala Asn Asn Ala Ala Ile Val Leu Gln Leu Pro Gln Gly Thr Thr Leu
        35                  40                  45

Pro Lys Gly Phe Tyr Ala Glu Gly Ser Arg Gly Gly Ser Gln Ala Ser
    50                  55                  60

Ser Arg Ser Ser Ser Arg Ser Arg Asn Ser Ser Arg Asn Ser Thr Pro
65                  70                  75                  80

Gly Ser Ser Arg Gly Thr Ser Pro Ala Arg Met Ala Gly Asn Gly Gly
                85                  90                  95

Asp Ala Ala Leu Ala Leu Leu Leu Leu Asp Arg Leu Asn Gln Leu Glu
            100                 105                 110

Ser Lys Met Ser Gly Lys Gly Gln Gln Gln Gln Gly Gln Thr Val Thr
        115                 120                 125

Lys Lys Ser Ala Ala Glu Ala Ser Lys Lys Pro Arg Gln Lys Arg Thr
    130                 135                 140

Ala Thr Lys Ala Tyr Asn Val Thr Gln Ala Phe Gly Arg Arg Gly Pro
145                 150                 155                 160

Glu Gln Thr Gln Gly Asn Phe Gly Asp Gln Glu Leu Ile Arg Gln Gly
                165                 170                 175

Thr Asp Tyr Lys His Trp Pro Gln Ile Ala Gln Phe Ala Pro Ser Ala
            180                 185                 190

Ser Ala Phe Phe Gly Met Ser Arg Ile Gly Met Glu Val Thr Pro Ser
        195                 200                 205

Gly Thr Trp Leu Thr Tyr Thr Gly Ala Ile Lys Leu Asp Asp Lys Asp
    210                 215                 220

Pro Asn Phe Lys Asp Gln Val Ile Leu Leu Asn Lys His Ile Asp Ala
225                 230                 235                 240

Tyr Lys Thr Phe Pro Pro Thr Glu Pro Lys Lys Asp Lys Lys Lys Lys
                245                 250                 255

Ala Asp Glu Thr Gln Ala Leu Pro Gln Arg Gln Lys Lys Gln Gln Thr
            260                 265                 270

Val Thr Leu Leu Pro Ala Ala Asp Leu Asp Asp Phe Ser Lys Gln Leu
        275                 280                 285

Gln Gln Ser Met Ser Ser Ala Asp Ser Thr Gln Ala
    290                 295                 300

<210> 4
<211> 680
<212> PRT
<213> Artificial Sequence

<220>
<223> SARS-CoV-2 Spike-Protein, C-terminally his-tagged, as after
      cleavage of signal peptide

<400> 4

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
        180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly

            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr
465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg Leu Glu
            660                 665                 670

His His His His His His His His
        675                 680

## Description

The present invention relates to method for determining whether a subject produces antibodies as a result of administration of a vaccine for distinguishing whether a subject produces antibodies as a result of administration of a vaccine or as a result of a previous corona virus infection, comprising the step detecting in a sample comprising antibodies from said subject the absence or presence of an antibody to SEQ ID NO1 and the absence or presence of an antibody to SEQ DI NO2.

At the end of 2019, a rising number of pneumonia patients with unknown pathogen emerged from Wuhan, the capital of Hubei province, China, to nearly the entirety of China. A novel coronavirus was isolated and based on its phylogeny, taxonomy and established practice, the Coronavirus Study Group (CSG) recognized it as a sister to severe acute respiratory syndrome coronaviruses (SARS-CoVs) and labeled it as severe acute respiratory syndrome coronavirus 2 (SARS-CoV-2).

Although SARS-CoV-2 is generally less pathogenic than SARS-CoV and Middle East respiratory syndrome coronavirus (MERS-CoV), it has a relatively high transmissibility. Since symptoms may be mild and may be confused with a cold, there is the danger that patients are unaware that they have been infected and may help the virus spread further.

In view of the massive impact of SARS-CoV-2 on economy and healthcare systems, there is considerable interest in being able to distinguish non-immunized healthy, immunized health and infected subjects. In particular, immunized subjects, vaccinated or previously infected, may be less contagious than non-immunized subjects. They may be able to return to regular work sooner than non-immunized patients.

Various projects aim to develop vaccines, based on a variety of technologies, including adenovirus vectors, RNA, inactivated SARS-CoV-2 virus and isolated SARS-CoV-2 components, in particular Nucleocapsid protein and S1.

One of the advantages associated with the latter approach is the low infection risk owing to the absence of any pathogens or derivatives thereof and preliminary data suggests that such an approach may yield an immunization. If such vaccines are to be developed, it is important to be able to test their efficacy, for example in preclinical trials on laboratory animals and clinical trials on humans.

Therefore, the problem underlying the present invention is to provide a method for such a distinction, in particular where a subject may have been vaccinated by administration of a composition comprising the S1 protein from SARS-CoV-2.

The problems are solved by the subject matter of the independent and dependent claims.

In a first aspect, the problem underlying the present invention is solved by a method for determining whether a subject produces antibodies as a result of administration of a vaccine, comprising the step detecting in a sample comprising antibodies from said subject
a) the absence or presence of an antibody to SEQ ID NO1 and
b) the absence or presence of an antibody to SEQ DI NO2.

In a second aspect, the problem underlying the present invention is solved by a method for distinguishing whether a subject produces antibodies as a result of administration of a vaccine or as a result of a previous corona virus infectio, preferably SARS-CoV-2 infection comprising the step detecting in a sample comprising antibodies from said subject
a) the absence or presence of an antibody to SEQ ID NO1 and
b) the absence or presence of an antibody to SEQ DI NO2.

In a preferred embodiment, the antibody to SEQ ID NO2 is an antibody to SEQ ID NO3.

In a preferred embodiment, the antibody to SEQ ID NO1 is an IgA, IgG or IgM class antibody, preferably IgG or IgM, more preferably IgG.

In a preferred antibody, the antibody to SEQ ID NO2 is an IgA, IgG or IgM class antibody, preferably IgM or IgG, more preferably IgG.

In a preferred embodiment, any antibody is detected using a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques.

In a preferred embodiment, the presence or absence of the antibody in step a) is monitored over at least one, preferably two, more preferably four weeks.

In a third aspect, the problem underlying the present invention is solved by use of a first polypeptide comprising SEQ ID NO1 or a variant thereof and a second polypeptide comprising SEQ ID NO2 or a variant thereof for determining whether a subject produces antibodies as a result of administration of a vaccine.

In a third aspect, the problem underlying the present invention is solved by a use of a first polypeptide comprising SEQ ID NO1 or a variant thereof and a second polypeptide comprising SEQ ID NO2 or a variant thereof for distinguishing whether a subject produces antibodies as a result of administration of a vaccine or as a result of a previous corona virus infection, preferably SARS-CoV-2 infection.

In a preferred embodiment, the first and the second polypeptide are coated on a diagnostically useful solid carrier.

In a preferred embodiment, the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

In a preferred embodiment, the method is for determining the efficacy of a vaccine candidate comprising a polypeptide comprising SEQ ID NO1 or a variant thereof.

In a preferred embodiment, the vaccine does not comprise SEQ ID NO2 or an immunogenic variant thereof, preferably an immunogenic variant.

In a preferred embodiment, it is monitored whether an antibody to SEQ ID NO1 (S1) is present, preferably for at least one, two, three, four, five or six weeks, preferably four weeks.

In a preferred embodiment, it is monitored whether an antibody to SEQ ID NO2 is present, preferably for at least one, two, three, four, five or six weeks, preferably four weeks.

In a preferred embodiment, the sample is a blood sample, preferably from the group comprising serum, plasma and whole blood.

The present invention centers around determining the immune status of a subject, more specifically whether the subject has been infected previously, but is now health, has been vaccinated or had no previous exposure to SARS-CoV-2 or any component of it.

The subject is from a species that may be infected by SARS-CoV-2 and may develop an immune response against it. In a preferred embodiment, the subject is a mammal or a bird, more preferably a mammal, more preferably a mammal from the group comprising goat, rabbit, horse, mouse, rat, donkey, bat, camel, primate and human, most preferably human.

The sample is from such a subject. It may be obtained as part of a trial using animals to check the immunogenicity or efficacy of a vaccine or it may be obtained from a human subject in a clinical trial, where the titers of antibodies are preferably monitored. In a preferred embodiment, it comprises a set of representative antibodies from the subject. In another preferred embodiment, it comprises a fraction of the sample enriched with regard to antibodies, preferably with regard to a class of antibodies, for example IgG, IgA and IgM. In a preferred embodiment, the sample is a blood sample, preferably selected from the group comprising whole blood, serum or plasma. The subject may be a laboratory animal.

In a preferred embodiment, the presence or absence of an antibody to SEQ ID NO1 is determined, preferably an antibody from the group of classes comprising IgG, IgA and IgM. The class of the antibody to SEQ ID NO1 may be IgG. The class of the antibody to SEQ ID NO1 may be IgM. The class of the antibody to SEQ ID NO1 may be IgA.

In a preferred embodiment, the presence or absence of an antibody to SEQ ID NO2 is determined, preferably an antibody from the group of classes comprising IgG, IgA and IgM. The class of the antibody to SEQ ID NO2 may be IgG. The class of the antibody to SEQ ID NO2 may be IgM. The class of the antibody to SEQ ID NO2 may be IgA. In a more preferred embodiment, the presence or absence of an antibody to SEQ ID NO3 is determined, preferably an antibody from the group of classes comprising IgG, IgA and IgM. The class of the antibody to SEQ ID NO3 may be IgG. The class of the antibody to SEQ ID NO3 may be IgM. The class of the antibody to SEQ ID NO3 may be IgA.

Preferably antibodies of the same class against SEQ ID NO1 and SEQ ID NO2 are detected, preferably IgA, IgM or IgG, more preferably IgG.

If an antibody to SEQ ID NO1 is present in the sample and an antibody to SEQ DI NO2 is absent, this indicates an increased likelihood that the subject has been successfully vaccinated with a vaccine comprising S1 protein from SARS-CoV-2 or a variant thereof, which may comprise a polypeptide comprising SEQ ID NO2 or a variant thereof. Likewise, If an antibody to SEQ ID NO2 is present in the sample and an antibody to SEQ DI NO1 is absent, this indicates an increased likelihood that the subject has been successfully vaccinated with a vaccine comprising nucleocapsid protein from SARS-CoV-2 or a variant thereof, which may comprise a polypeptide comprising SEQ ID NO2, preferably SEQ ID NO3 or a variant thereof.

Alternatively, if the level of antibodies to SEQ ID NO1 increases, while the level of antibodies to SEQ ID NO2 does not increase, this indicates an increased likelihood that the subject has been successfully vaccinated with a vaccine comprising SEQ ID NO1 or a variant thereof rather than having been exposed to full SARS-CoV-2, for example by way of an infection.

If both an antibody to SEQ ID NO1 and an antibody to SEQ ID NO2 are detectable or their levels increase, this suggests that the patient is or was infected with SARS-CoV-2. If the patient has symptoms and/or an IgM and/or IgA class antibodies can be detected or if the levels of IgM and/or IgA and/or IgG class antibodies are increasing, this suggests an active infection. The absence of symptoms and/or IgM and/or IgA class antibodies suggests a past infection. A PCR-based diagnostic assay for SARS-CoV-2 may be run in addition, in particular if a very early stage of the infection is suspected.

In a preferred embodiment, the presence or absence of symptoms typical for SARS-CoV and SARS-CoV-2 are taken into account to distinguish whether the subject suffers or has suffered from SARS or SARS-CoV2.

In a preferred embodiment, the presence or absence of an antibody to SEQ ID NO1 is detected, preferably monitored for at least 1, 2, 3 or 4 weeks. The presence of an antibody to SEQ ID NO2, preferably SEQ ID NO3 may be determined only if an antibody to SEQ ID NO1 is detected.

Antibodies to SEQ ID NO1 may be detectable before antibodies to SEQ ID NO2 are. In a preferred embodiment, the presence of antibodies to SEQ ID NO2 is monitored for at least 1, 2, 3 or 4 weeks after a negative result or determined again at at least one more later time point to increase the reliability of the result. It has to be borne in mind that IgA and IgM class antibodies emerge before IgG class antibodies do.

In a preferred embodiment, a vaccine has been administrated to the subject, preferably at least 1, 2, 3, 4, 5, 6, 7, 10, 14 days prior to the detection of antibodies according to the present invention.

Preferably the efficacy of a vaccine to be developed is assessed by the level of antibody or antibodies detected, for example IgA, IgM or IgG class antibodies, preferably IgG class. A neutralization test may be carried out in addition to determine whether the antibodies produced are neutralizing antibodies. If a vaccine or candidate vaccine comprising SEQ ID NO1 or a variant thereof has been administrated and antibodies to SEQ ID NO1 are detectable, but not to SEQ ID NO2, then the vaccine or vaccine candidate is capable of immunizing the subject, more preferably trigger an immune response with antibodies reactive to SARS-CoV-2. If antibodies to SEQ ID NO1 are not detectable after a period of time sufficient to allow for the production of such antibodies, then the vaccine or candidate vaccine is ineffective.

In a preferred embodiment, the vaccine is a composition comprising SEQ ID NO1 or a variant thereof which composition triggers the production of antibodies in the subject, preferably to SEQ ID NO1, only, *i.e.* not to other SARS-CoV-2 proteins, in particular SEQ ID NO2 or SEQ ID NO3 or variants thereof, preferably immunogenic variants. The composition may comprise an adjuvans and a physiologically compatible buffer such as PBS. In a preferred embodiment, the vaccine does not comprise SEQ ID NO2 or SEQ ID NO3 or a variant thereof at a level that would be able to trigger the production of antibodies to SEQ ID NO2. The generation of such pharmaceutical compositions and adjuvants and delivery systems are described in Vaccinology Principles and Practice by Morrow/Sheikh/Schmidt/Davies, Wiley.

Preferably two or more or three or more shots of vaccine are administered to the subject. A vaccination may be recognized preferably only after two or three or more weeks, after a detectable level of antibodies, initially IgA or IgM class antibodies, followed by IgG class antibodies, is produced by the subject.

In a preferred embodiment, the method or use according to the present invention comprises the step providing a polypeptide comprising SEQ ID NO 1 and a variant thereof and a polypeptide comprising SEQ ID NO2, preferably SEQ ID NO3 and a variant thereof, both polypeptides preferably coated on a diagnostically useful carrier or separate diagnostically useful carriers, and a sample from a subject. The carrier or each carrier may then be contacted with the sample under conditions allowing for binding of any antibodies to SEQ ID NO1, SEQ ID NO2 or SEQ ID NO3. The sample may then be removed and the carrier with may be washed to remove any remaining sample. A secondary antibody or similar reagent or means binding to the antibody and carrying a detectable label may then be contacted with the carrier under conditions allowing formation of a complex between any bound antibody and the secondary antibody. The carrier may be washed then to remove non-bound secondary antibody. Finally, the presence or absence of the antibody is detected by checking whether the secondary antibody may be detected.

In a preferred embodiment, the diagnostically useful carrier is a line blot (Raoult, D., and Dasch, G. A. (1989), The line blot: an immunoassay for monoclonal and other antibodies. Its application to the serotyping of gram-negative bacteria. J. Immunol. Methods, 125 (1-2), 57-65; WO2013041540). In a preferred embodiment, the term "line blot", as used herein refers to a test strip, more preferably membrane-based, that has been coated with one or more means for specifically capturing an antibody, preferably each of them is a polypeptide. If two or more means are used, they are preferably spatially separated on the carrier. Preferably, the width of the bands is at least 30, more preferably 40, 50, 60, 70 or 80 % the width of the test strip. The line blot may comprise one or more control bands for confirming that it has been contacted with sample sufficiently long and under adequate conditions, in particular in the presence of human serum, or with a secondary antibody, respectively.

In another preferred embodiment, the diagnostically useful carrier is a bead. Various beads for numerous applications are commercially available, mainly based on carbohydrate, for example sepharose or agarose, or plastic. They contain active or activatable chemical groups such as a carboxyl or ester group, which can be utilized for the immobilization of a means for specifically capturing an antibody. Preferably, the beads are beads having an average diameter of from 0.1 µm to 10 µm, from 0.5 µm to 8 µm, from 0.75 µm to 7 µm or from 1 µm to 6 µm. The beads can be coated with the means for specifically capturing an antibody directly or *via* affinity ligands, for example biotin or glutathione and streptavidin and GST, respectively. For example, the bead may be coated with biotin or glutathione and the antigen may be fused with streptavidin or glutathione-S-transferase or a variant thereof, respectively. Preferably, the bead is provided in the form of an aqueous suspension having a bead content of from 10 to 90%, preferably from 20 to 80%, preferably from 30 to 70%, more preferably from 40 to 60% (w/w). In a preferred embodiment, the bead is coated with a first detectable marker, for example a fluorescent dye.

In a particularly preferred embodiment, the beads are paramagnetic beads, which can be easily concentrated on a surface with the aid of a magnet. For this purpose, commercial paramagnetic beads usually contain a paramagnetic mineral, for example iron oxide. A multiplicity of suitable paramagnetic beads is commercially available. A bead may be labeled with a detectable label.

In another preferred embodiment, the carrier is a microtiter plate, preferably comprising at least eight wells that may be used for ELISA. Two or more microtiter plates may be used. At least one of the wells is coated with the polypeptide comprising SEQ ID NO1 or a variant thereof, and at least one other well is coated with the polypeptide comprising SEQ ID NO2 or a variant thereof. At least 3, preferably 4, more preferably 5 calibrators are provided that comprise an antibody to an antigen, preferably both antigens from the group comprising SEQ ID NO1 and SEQ ID NO2 or a variant thereof, at defined concentrations and may be used to set up a calibration curve for semi-quantitative analysis. When the inventive method is carried out, the calibrators may be processed and developed in parallel to the samples. A secondary antibody comprising a detectable label such as an enzymatically active label may be provided, for example a label having horse radish peroxidase activity or alkaline phosphatase activity or an enzyme capable of chemiluminescence.

In another preferred embodiment, the carrier is a microarray. In a preferred embodiment, the term "microarray", as used herein, refers to a chip spotted with a variety of spatially separate antigens, preferably at least 20, preferably 30, 40, 50, 80 or 100. They include SEQ ID NO1 and SEQ ID NO2 or a variant thereof.

The polypeptide may be provided in any form and at any degree of purification, from tissues, fluids or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from cells, more preferably from prokaryotic or eukaryotic, preferably mammalian cells. A glycosylated form of the polypeptide may be used.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

The inventive teachings provide a kit. Such a kit is a container that comprises specific reagents required to practice the inventive method, in particular the diagnostically useful carrier according to the present invention, optionally in addition to one or more solutions or reagents required to practice the inventive method, preferably selected from or all from the group comprising sample dilution buffer, washing buffer and buffer comprising a means or reagent for detecting any specifically captured antibody, such as a secondary antibody which may comprise a detectable label and optionally a means for detecting the latter.

Furthermore, it may comprise instructions detailing how to use the kit. It may comprise the diagnostically useful carrier for contacting the inventive polypeptide with a sample from a subject, preferably selected from the group comprising a microtiter plate, two or more beads and a line blot. Furthermore, the kit may comprise two positive controls, for example a recombinant or polyclonal antibody known to bind to to SEQ ID NO1 and another antibody known to bind to SEQ ID NO2, preferably SEQ ID NO3, and a negative control, for example a protein having no detectable affinity to SEQ ID NO1 or SEQ ID NO2 such as bovine serum albumin. The positive control may be a diluted sample from a patient who had been exposed to SARS-CoV-2. A negative control may be a diluted sample from a healthy blood donor without antibodies to SARS-CoV-2. Another positive sample representing a successfully vaccinated subject may be a sample from such a subject or a solution comprising an antibody to SEQ ID NO1 or SEQ ID NO2, but not both. Finally, such a kit may comprise one or more standard solutions, also referred to as calibrators, comprising one or more antibodies binding to SEQ ID NO1, and may comprise one or more standard solutions, also referred to as calibrator, comprising one or more antibodies binding to SEQ ID NO2, wherein the absolute or relative concentration of the antibody in each standard solution is preferably known. The concentration in the standard solutions may differ such that a concentration range is covered for a calibration curve. For example, the relative concentration of the respective antibody in two standard solutions may be 1:2, 1:5, 1:10, 1:20, 1:50 or 1:100 or more. In a more preferred embodiment, the antibody has a constant region or variant thereof or binding affinity to secondary antibodies like the antibody to be detected and/or any secondary antibody used for the detection binds to said antibody and the antibody to be detected. The antibody may be a monoclonal antibody, preferably a hybrid comprising a human IgA, IgM or IgG, preferably IgG constant region or a variant thereof binding to secondary antibodies binding to human IgA, IgM or IgG, preferably IgG antibodies. The kit may comprise a polypeptide comprising SEQ ID NO1 or a variant thereof, which polypeptide may optionally comprise a detectable label. The kit may comprise a polypeptide comprising SEQ ID NO2 or a variant thereof, which polypeptide may optionally comprise a detectable label. The kit may also comprise a washing solution. The kit may comprise a suitable water-tight vessel for contacting the diagnostically useful carrier with the sample in the presence of other liquids such as a reaction buffer. For example, a line blot may be provided in or in combination with an incubation tray, or a microtiter plate may be provided. Suitable vessels are described in the state of the art, for example EP3025780 or EP3025779. The kit may contain reagents for implementing a method according to the present invention, for example the method according to the 8^{th} aspect. Calibrators and their use are described in in The Immunoassay Handbook, 3rd edition, by David Wild, Elsevier 2005, in particular Chapter 9. Such a kit may be taken for the uses according to the present invention.

The antibody to be detected binds preferably specifically to SEQ ID NO1 or SEQ ID NO2, preferably SEQ ID NO3. Specific binding preferably means that the binding reaction is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7 or ELISA as described in the examples, preferably ELISA.

According to the present invention, a medical or diagnostic device such as the diagnostically useful carrier may be prepared by expressing a recombinant variant of SEQ ID NO1 or SEQ ID NO2, preferably SEQ ID NO3 comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, Faktor Xa or Enteropeptidase. Suitable linkers are part of vectors, for example pET vector series (Novagen).

The method and reagents according to the present invention may also be used for screening the potency and the usefulness of an antiviral drug, preferably in a library of drug candidates.

The method and reagents according to the present invention may also be used for screening whether donated blood is contaminated with coronavirus.

The methods and reagents according to the present invention may also be used for screening whether donated blood comprises antibodies to SARS-CoV-2 and may be used to prepare a medicament used to treat a patient suffering from SARS-CoV-2 and who may benefit from the administration of such antibodies. In particular, blood comprising an antibody to SEQ ID NO1 and an antibody to SEQ ID NO2 is from a patient having been exposed to SARS-CoV-2. This may have been the results of an active infection or a vaccination using an inactivated virus or SEQ ID NO1 and SEQ ID NO2 or a variant thereof. In a preferred embodiment, blood from a patient may be used to treat another patient suffering from a severe SARS-CoV-2 infection or to prepare a medicament for such a treatment. In another preferred embodiment, blood from a subject having been vaccinated with a vaccine comprising SEQ ID NO1 and SEQ ID NO2 or a variant thereof, preferably an inactivated virus may be used to treat another patient suffering from a severe SARS-CoV-2 infection or to prepare a medicament for such a treatment.

The teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to or a polypeptide comprising said fragment, more specifically to one or more amino acid or nucleic acid sequences which are, relative to the full-length sequence, truncated at one or both termini by one or more amino acids. Such a fragment comprises or encodes for a peptide having at least 10, 15, 25, 50, 75, 100, 150, 200, 250, 300, 400, 500 or 600 successive amino acids of the original sequence or for a variant thereof.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

SARS-CoV-2-related publications on specific amino acid sequences such as Beal et al. may aid the skilled one in designing variants (Beal, J., Mitechell, T., Wyschogrod, W., Manthey, J, and Clore, A. (2020) Highly Distinguished Amino Acid Sequences of 2019-nCoV (Wuhan Coronavirus) doi: https://doi.org/10.1101/2020.01.31.929497, as well as publications relating to SARS-CoV, for example Hua, R., Zhou, Y., Wang, Y., Hua, Y and Tong, T. (2004) Identification of two antigenic epitopes on SARS-CoV spike protein, BBR 319, 929-935, wherein homologous epitopes may be found and SARS-CoV-2 epitopes be identified on account of their homology. Dahlke, C., Heidepriem, J., Kobbe, R., Santer, R., Koch, T., Fathi, A., Ly, M. L., Schmiedel, S., Seeberger, P. H., and Loeffler, F. (2020), medRxiv, https://doi.org/10.1101/2020.04.14.20059733 contacted a peptide microarray with patient samples and identified reactive epitopes.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, affinity tags, other antigens and the like. For example, SEQ ID NO4 is a fusion protein according to the present invention.

The variant of the polypeptide has biological activity. In a preferred embodiment such biological activity is the ability to bind to the respective antibody such as an antibody to SEQ ID NO1 or an antibody to SEQ ID NO2, preferably SEQ ID NO3. In a preferred embodiment it comprises an epitope having the ability or has itself the ability to bind to the antibody to be detected, preferably from a sample from a patient suffering from SARS-CoV-2, wherein more preferably the epitope comprises a sequence comprising a stretch of at least 5, 6, 7 or 8 amino acid residues from the wildtype sequence, more specifically SEQ ID NO1 or an antibody to SEQ ID NO2. More preferably, it does not bind specifically to homologues from other coronaviruses, preferably coronaviruses other than SARS-CoV, more preferably from the group comprising NL63, 229E, OC43, HKU1.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. Preferably the test format is an ELISA and a microtiter plate comprising wells is used as a diagnostically useful carrier. In a preferred embodiment, the term "presence" of an antibody, as used herein, means that the antibody is detectable using such a method, preferably a method selected from the group comprising enzyme immunoassays, more preferably colorimetric assays or chemiluminescence immunoassays, most preferably colorimetric assays based on line blot and detection using alkaline phosphatase activity as described in the examples.

Preferably the first and the second polypeptide or a variant thereof is immobilized on a solid phase of the carrier. It may be directly immobilized on the solid phase when contacted with the sample, but a competitive assay, a capture bridge assay, an immunometric assay, a class-specific second antibody on the solid phase, a class capture assay, direct or indirect may also be used. The principle of each of these formats is detailed in The Immunoassay Handbook, 3rd edition, edited by David Wild, Elsevier, 2005. More preferably, the solid phase is a test strip or a well of a microtiter plate for ELISA or a bead, preferably a well of a microtiter plate for ELISA. The first and the second polypeptide may be on the same or on different carriers.

In a preferred embodiment, the first and the second polypeptide are spatially separate such that a signal indicating the presence of an antibody to SEQ ID NO1 and a signal indicating the presence of an antibody to SEQ ID NO2, preferably SEQ ID NO3 can be distinguished. For example, the first and the second polypeptides may be on different beads, on different wells of one or more microtiter plates or spatially separate spots on a membrane such as a blot membrane, for example Western blot, dot blot or line blot. The membrane may optionally be on a microtiter plate well.

In a preferred embodiment, a vaccine comprising SEQ ID NO1 or a variant thereof is provided and preferably administered to a subject, preferably by injection.

In a preferred embodiment, the method or use comprises step c) providing the vaccine for administration to a subject or patient, preferably followed by administration to a subject or patient. For example, the antigen may be expressed in a large scale and transferred in a sterile liquid, optionally comprising an adjuvans, into a syringe for injection to the patient or another device for administration. A kit comprising such a device and instructions how to use it may be manufactured or provided. The skilled person knows how to manufacture vaccines and this is described in the state of the art, for example in Wen, E. P., Ellis, R., Pujar, N. S.: Vaccine Development and Manufacturing, Wiley, 2015.

The present invention comprises a range of polypeptide sequences, more specifically

SEQ ID NO1 (SARS-CoV-2 S1 Spike-Protein)

VNLTTRTQLPPAYTNSFTRGVYYPDKVFRSSVLHSTQDLFLPFFSNVTWFHAIHVSGTNGT
KRFDNPVLPFNDGVYFASTEKSNIIRGWIFGTTLDSKTQSLLIVNNATNVVIKVCEFQFCNDP
FLGVYYHKNNKSWMESEFRVYSSANNCTFEYVSQPFLMDLEGKQGNFKNLREFVFKNIDG
YFKIYSKHTPINLVRDLPQGFSALEPLVDLPIGINITRFQTLLALHRSYLTPGDSSSGWTAGAA
AYYVGYLQPRTFLLKYNENGTITDAVDCALDPLSETKCTLKSFTVEKGIYQTSNFRVQPTESI
VRFPNITNLCPFGEVFNATRFASVYAWNRKRISNCVADYSVLYNSASFSTFKCYGVSPTKLN
DLCFTNVYADSFVIRGDEVRQIAPGQTGKIADYNYKLPDDFTGCVIAWNSNNLDSKVGGNY
NYLYRLFRKSNLKPFERDISTEIYQAGSTPCNGVEGFNCYFPLQSYGFQPTNGVGYQPYRV
VVLSFELLHAPATVCGPKKSTNLVKNKCVNFNFNGLTGTGVLTESNKKFLPFQQFGRDIAD
TTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRAR

SEQ DI NO2 SARS-CoV-2 nucleoprotein (full length) (data base code:
YP_009724397.2)

MSDNGPQNQRNAPRITFGGPSDSTGSNQNGERSGARSKQRRPQGLPNNTASWFTALTQ
HGKEDLKFPRGQGVPINTNSSPDDQIGYYRRATRRIRGGDGKMKDLSPRWYFYYLGTGPE
AGLPYGANKDGIIWVATEGALNTPKDHIGTRNPANNAAIVLQLPQGTTLPKGFYAEGSRGG
SQASSRSSSRSRNSSRNSTPGSSRGTSPARMAGNGGDAALALLLLDRLNQLESKMSGKG
QQQQGQTVTKKSAAEASKKPRQKRTATKAYNVTQAFGRRGPEQTQGNFGDQELIRQGTD
YKHWPQIAQFAPSASAFFGMSRIGMEVTPSGTWLTYTGAIKLDDKDPNFKDQVILLNKHIDA
YKTFPPTEPKKDKKKKADETQALPQRQKKQQTVTLLPAADLDDFSKQLQQSMSSADSTQA

SEQ ID NO3 SARS-CoV-2 nucleoprotein (118-419), basis for designing the polypeptide
used in Example 1
GLPYGANKDGIIWVATEGALNTPKDHIGTRNPANNAAIVLQLPQGTTLPKGFYAEGSRGGS
QASSRSSSRSRNSSRNSTPGSSRGTSPARMAGNGGDAALALLLLDRLNQLESKMSGKGQ
QQQGQTVTKKSAAEASKKPRQKRTATKAYNVTQAFGRRGPEQTQGNFGDQELIRQGTDY
KHWPQIAQFAPSASAFFGMSRIGMEVTPSGTWLTYTGAIKLDDKDPNFKDQVILLNKHIDAY
KTFPPTEPKKDKKKKADETQALPQRQKKQQTVTLLPAADLDDFSKQLQQSMSSADSTQA

SEQ ID NO4 (SARS-CoV-2 Spike-Protein, C-terminally his-tagged, as after cleavage of
signal peptide, used in the examples)
VNLTTRTQLPPAYTNSFTRGVYYPDKVFRSSVLHSTQDLFLPFFSNVTWFHAIHVSGTNGT
KRFDNPVLPFNDGVYFASTEKSNIIRGWIFGTTLDSKTQSLLIVNNATNVVIKVCEFQFCNDP
FLGVYYHKNNKSWMESEFRVYSSANNCTFEYVSQPFLMDLEGKQGNFKNLREFVFKNIDG
YFKIYSKHTPINLVRDLPQGFSALEPLVDLPIGINITRFQTLLALHRSYLTPGDSSSGWTAGAA
AYYVGYLQPRTFLLKYNENGTITDAVDCALDPLSETKCTLKSFTVEKGIYQTSNFRVQPTESI
VRFPNITNLCPFGEVFNATRFASVYAWNRKRISNCVADYSVLYNSASFSTFKCYGVSPTKLN
DLCFTNVYADSFVIRGDEVRQIAPGQTGKIADYNYKLPDDFTGCVIAWNSNNLDSKVGGNY
NYLYRLFRKSNLKPFERDISTEIYQAGSTPCNGVEGFNCYFPLQSYGFQPTNGVGYQPYRV
VVLSFELLHAPATVCGPKKSTNLVKNKCVNFNFNGLTGTGVLTESNKKFLPFQQFGRDIAD
TTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRARLEHHHHHHH
H

### Example 1: Preparation of antigen

To prepare SEQ ID NO4, cell culture supernatant was adjusted to 5 mmol/l tris chloride pH 8.0, 164 mmol/l sodium chloride, 50 mmol/l magnesium chloride, 20 mmol/l imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/l tris chloride pH 8.0, 300 mmol/l sodium chloride, 20 mmol/l imidazole and eluted by increasing the imidazole concentration to 150 mmol/l. All fractions containing SEQ ID NO3 were pooled and concentrated by ultrafiltration (VivaSpin, Sartorius, Göttingen, Germany). The final preparation was stored at -80°C until further use.

The final protein preparation of SEQ ID NO4 was treated with or without 16 mmol/l dithiotreitol and incubated at 70°C or at room temperature for 10 minutes, followed by SDS gel electrophoresis and Coomassie staining.

Protein identity was verified by mass spectrometry.

### Example 2: Vaccination studies

A healthy subject received on day 1 an injection into the musculus quadriceps of 12.86 µg recombinant S1 protein in physiological PBS (SEQ ID NO4). Alum adjuvans (Twinrix for adults, EMRA-MED Arzneimittel GmbH) was applied according to the manufacturer's instructions. On days 9, 21 and 28, the subject received an injection of another 12.86 µg S1 protein in physiological PBS buffer and 10 µl Imject alaun adjuvans in 500 µl sodium chlorid solution.

Blood samples were obtained on days 10, 23 and 29.

The presence of IgG and IgA antibodies to SARS-CoV-2 S1 and nucleoprotein was determined in serum samples from the healthy subject using serological kits (EUROIMMUN Medizinische Labordiagnostika AG, El 2606-9601 A, El 2606-9601 G, El 2606-9601-2 G) according to the manufacturer's instructions.

As controls, samples from healthy blood donors and from patients suffering from SARS-CoV-2 infection were used.

**Table 1: Antibodies to SARS-CoV-2 antigens in a subject vaccinated using S1 protein**

| Day | IgA (S1) | IgG (S1) | IgG (NCP) | IgM (NCP) |
|---|---|---|---|---|
| | (Cut off: <0.8) | (Cut off: <0.8) | (Cut off: <0.8) | (Cut off: <0.8) |
| 10 | 0.5 | 0.5 | - | - |
| 23 | 1.0 | 0.2 | - | - |
| 29 | 3.5 | 6.2 | 0.6 | 0.7 |

In two patients suffering from SARS-CoV-2 infection (positive controls), samples obtained 17 and 19 days after the onset of symptoms (usually 5-6 days after the infection). Antibody titers are shown in Table 2.

**Table 2: Antibodies to SARS-CoV-2 antigens in two patients suffering from SARS-CoV-2 infection**

| Day | IgA (S1) | IgG (S1) | IgG (NCP) | IgM (NCP) |
|---|---|---|---|---|
| | (Cut off: <0.8) | (Cut off: <0.8) | (Cut off: <0.8) | (Cut off: <0.8) |
| Patient 1 | 2,6 | 1,8 | 1,7 | 1,3 |
| 1,3 | 1.3 | 6.3 | 3.5 | 11.4 |

In four healthy subjects who did not receive any vaccination (negative controls), samples were obtained. Antibody titers are shown in Table 3.

**Table 3: Antibodies to SARS-CoV-2 antigens in healthy subjects**

| Day | IgA (S1) | IgG (S1) | IgG (NCP) | IgM (NCP) |
|---|---|---|---|---|
| | (Cut off: <0.8) | (Cut off: <0.8) | (Cut off: <0.8) | (Cut off: <0.8) |
| Patient 1 | 0,0 | 0,1 | 0,0 | 0,5 |
| Patient 2 | 0,2 | 0,3 | 0,1 | 0,4 |
| Patient 3 | 0,1 | 0,1 | 0,1 | 0,2 |
| Patient 4 | 0,1 | 0,1 | 0,1 | 0,1 |

These results show that vaccinated and infected subjects can be distinguished. Antibodies to the S1 antigen comprised in the vaccine can be detected in both, but antibodies to the nucleocapsid protein only in infected patients, but not vaccinated subjects.

## Claims

1. A method for determining whether a subject produces antibodies as a result of administration of a vaccine, comprising the step detecting in a sample comprising antibodies from said subject
a) the absence or presence of an antibody to SEQ ID NO1 and
b) the absence or presence of an antibody to SEQ DI NO2.

2. A method for distinguishing whether a subject produces antibodies as a result of administration of a vaccine or as a result of a previous corona virus infection, preferably SARS-CoV-2 infection comprising the step detecting in a sample comprising antibodies from said subject
a) the absence or presence of an antibody to SEQ ID NO1 and
b) the absence or presence of an antibody to SEQ DI NO2.

3. The method according to any of claims 1 and 2, wherein the antibody to SEQ ID NO2 is an antibody to SEQ ID NO3.

4. The method according to any of claim 1 to 4, the antibody to SEQ ID NO1 is an IgA, IgG or IgM class antibody, preferably IgG or IgM, more preferably IgG.

5. The method according to any of claims 1 to 4, wherein the antibody to SEQ ID NO2 is an IgA, IgG or IgM class antibody, preferably IgM or IgG, more preferably IgG.

6. The method according to any of claims 1 to 5, wherein any antibody is detected using a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques.

7. The method according to any of claims 1 to 6, wherein the presence or absence of the antibody in step a) is monitored over at least one, preferably two, more preferably four weeks.

8. A use of a first polypeptide comprising SEQ ID NO1 or a variant thereof and a second polypeptide comprising SEQ ID NO2 or a variant thereof for determining whether a subject produces antibodies as a result of administration of a vaccine.

9. A use of a first polypeptide comprising SEQ ID NO1 or a variant thereof and a second polypeptide comprising SEQ ID NO2 or a variant thereof for distinguishing whether a subject produces antibodies as a result of administration of a vaccine or as a result of a previous corona virus infection, preferably SARS-CoV-2 infection.

10. The use according to any of claims 8 to 9, wherein the first and the second polypeptide are coated on a diagnostically useful solid carrier.

11. The use according to claim 10, wherein the carrier is selected from the group comprising a bead, a test strip, a microtiter plate, a microarray, a blot, preferably from the group comprising western blot, line blot and dot blot, a glass surface, a slide, a biochip and a membrane, and is most preferably a microtiter plate or a line blot.

12. The method according to any of claims 1 to 7, wherein the method is for determining the efficacy of a vaccine candidate comprising a polypeptide comprising SEQ ID NO1 or a variant thereof.

13. The use or method according to any of claims 1 to 12, wherein the vaccine does not comprise SEQ ID NO2 or an immunogenic variant thereof, preferably an immunogenic variant.

14. The method according to any of claims 1 to 7 and 12 to 13, wherein it is monitored whether an antibody to SEQ ID NO1 (S1) is present, preferably for at least one, two, three, four, five or six weeks, preferably four weeks.

15. The method according to any of claims 1 to 7 and 12 to 14 to, wherein it is monitored whether an antibody to SEQ ID NO2 is present, preferably for at least one, two, three, four, five or six weeks, preferably four weeks.

16. The method according to any of claims 1 to 7 and 12 to 15, wherein a vaccine comprising SEQ ID NO1 or a variant thereof is provided and preferably administered to a subject.

17. The method according to any of claims 1 to 7 and 12 to 16, wherein the method comprises step c) providing the vaccine for administration to a patient, preferably followed by administration to a patient.
